# EUROPÄISCHE PATENTSCHRIFT

(11) **EP 3 473 217 B1**
(45) Veröffentlichungstag und Bekanntmachung des Hinweises auf die Patenterteilung: **07.10.2020**
(21) Anmeldenummer: 18189352.0
(22) Anmeldetag: 16.08.2018
(51) Int. Cl.: A61F 9/007

(54) **VORRICHTUNG ZUM SCHNEIDEN UND ABSAUGEN VON GEWEBE AUS DEM MENSCHLICHEN ODER TIERISCHEN AUGE**
DEVICE FOR CUTTING AND ASPIRATING TISSUE FROM A HUMAN OR ANIMAL EYE
DISPOSITIF DE COUPE ET D'ASPIRATION DE TISSU DE L' OEIL HUMAIN OU ANIMAL

(30) Priorität: 17.10.2017 DE 102017218491
(43) Veröffentlichungstag der Anmeldung: 24.04.2019
(73) Patentinhaber: Geuder AG, 69126 Heidelberg (DE)
(72) Erfinder: Engel, Stefan, 69126 Heidelberg (DE); Schlund, Christopher, 69168 Wiesloch (DE)
(74) Vertreter: Patent- und Rechtsanwälte Ullrich & Naumann

(56) Entgegenhaltungen:
- EP-A1- 0 514 057
- EP-A1- 1 207 825
- US-A- 3 734 099

## Beschreibung

Die Erfindung betrifft eine Vorrichtung zum Schneiden und Absaugen von Gewebe aus dem menschlichen oder tierischen Auge, insbesondere zur Durchführung einer Vitrektomie oder eines Netzhautpeelings, mit einer äußeren Röhre und einer in der äußeren Röhre drehbaren inneren Röhre, wobei die äußere Röhre wenigstens eine seitliche Öffnung mit zumindest einer Außenschneidkante aufweist, wobei die innere Röhre mindestens eine seitliche Öffnung mit zumindest einer Innenschneidkante aufweist und wobei die Innenschneidkante und die Außenschneidkante beim Drehen der inneren Röhre gegenüber der äußeren Röhre schneidend zusammen wirken.

Grundsätzlich geht es hier um ein chirurgisches Schneidinstrument zur Entnahme von Gewebe aus dem Auge. Mit dem Instrument lässt sich das Gewebe - am bzw. im Auge - schneiden und vom Auge bzw. aus dem Auge heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt/abgesaugt wird. Auch lassen sich mit diesem Instrument Blut, Blutgerinnsel und bindegewebsähnliche Veränderungen sowie Bereiche der Netzhaut im Rahmen eines Netzhautpeelings entfernen.

Zum Stand der Technik sei lediglich beispielhaft auf die DE 10 2010 050 337 A1 verwiesen. Aus dieser Druckschrift ist eine Vorrichtung bekannt, wobei dort sowohl die äußere Röhre als auch die innere Röhre jeweils zwei seitliche Ausnehmungen mit Doppelschneidfunktion hat. Ähnliche Vorrichtungen sind aus der US 5,474,532, EP0514057, EP1207825, US3734099 und US 5,106,364 bekannt.

Bei diesen Vorrichtungen wird die innere Röhre in Axialrichtung alternierend verschoben, so dass die Schneidkanten der inneren Röhre und der äußeren Röhre schneidend zusammenwirken.

Des Weiteren sind aus der Praxis Vorrichtungen bekannt, bei welchen die innere Röhre gegenüber der äußeren Röhre drehbar ist. Auch bei einer solchen Ausgestaltung wirken die Schneidkanten der inneren Röhre und der äußeren Röhre schneidend zusammen. Dieses sog. "Drehschnittprinzip" hat den Vorteil, dass die Größe des Schneiden-Fensters, d.h. der seitlichen Öffnungen, nicht von dem Hub der Bewegung der inneren Röhre abhängig ist. Ein weiterer Vorteil liegt darin, dass durch den seitlichen Schnitt weitere bzw. andere Regionen in dem Auge mit der Vorrichtung bearbeitbar sind. Auch treten im Gegensatz zu einer Hubbewegung bei einer Drehbewegung der inneren Röhre geringere Vibrationen an den Schneidkanten auf.

Die aus der Praxis bekannten Vorrichtungen, die nach dem "Drehschnittprinzip" arbeiten, haben jedoch den Nachteil, dass sie äußerst kompliziert in der Konstruktion und somit teuer in der Herstellung und fehleranfällig sind.

Der vorliegenden Erfindung liegt daher die Aufgabe zugrunde, eine Vorrichtung zum Schneiden und Absaugen von Gewebe aus dem menschlichen oder tierischen Auge derart auszugestalten und weiterzubilden, dass mit konstruktiv einfachen Mitteln eine Drehbewegung der inneren Röhre gegenüber der äußeren Röhre realisierbar ist.

Erfindungsgemäß wird die voranstehende Aufgabe durch die Merkmale des Anspruches 1 gelöst. Danach ist eine Vorrichtung zum Schneiden und Absaugen von Gewebe aus dem menschlichen oder tierischen Auge dadurch gekennzeichnet, dass ein Drehelement einen sich zumindest bereichsweise radial erstreckenden Vorsprung zur Beaufschlagung mit Druckluft aufweist.

In erfindungsgemäßer Weise ist zunächst erkannt worden, dass die zugrundeliegende Aufgabe durch die Anordnung eines Drehelements auf besonders einfache Weise gelöst werden kann. Das Drehelement ist dabei derart ausgebildet, dass es bei Beaufschlagung mit Druckluft eine Drehbewegung ausführt. Da das Drehelement mittelbar oder unmittelbar mit der inneren Röhre gekoppelt ist, wird diese durch das Drehelement "mitgenommen" und führt somit eine Drehbewegung gegenüber der äußeren Röhre aus. Dadurch kann das zu entfernende Gewebe durch die seitlichen Öffnungen in die innere Röhre eingesaugt und von der Innenschneidkante und der Außenschneidkante abgetrennt werden. Aufgrund der äußerst einfachen Konstruktion ist eine sichere Funktionsweise der Vorrichtung ermöglicht.

Der Begriff "Druckluft" ist im weitesten Sinne zu verstehen. Dabei kann es sich um Luft oder ein beliebiges anderes Gas handeln. Des Weiteren kann es sich um einen relativ kurzen Druckluftimpuls bzw. Druckluftstoß oder eine im Wesentlichen konstante Beaufschlagung mit Druckluft handeln.

Unter dem Begriff "proximal" ist der während der Verwendung der Vorrichtung von dem Auge abgewandte Bereich zu verstehen. Der Begriff "distal" steht hingegen für den dem Auge zugewandten Bereich der Vorrichtung.

In vorteilhafter Weise können die innere Röhre und die äußere Röhre zumindest bereichsweise in und/oder an einem Gehäuse angeordnet sein, wobei das Gehäuse am proximalen Ende mit einem Gehäuseboden oder mit einem Gehäusedeckel verschlossen ist.

In weiter vorteilhafter Weise können das Gehäuse, beispielsweise der Gehäuseboden, und/oder der Gehäusedeckel mindestens einen Durchgang zur Beaufschlagung des Drehelementes mit Druckluft aufweisen. Der Durchgang ermöglicht es somit eine Fluidverbindung mit dem Gehäuseinneren herzustellen, so dass das Drehelement mit Druckluft beaufschlagbar ist. Dabei ist denkbar, dass der Durchgang an der Außenseite des Gehäuses bzw. des Gehäusedeckels als Anschluss für einen Druckluftschlauch ausgebildet ist.

Um das Drehelement auf besonders einfache und effektive Weise anzutreiben, kann der Durchgang derart ausgebildet sein, dass die Druckluft im Wesentlichen in Umfangsrichtung in das Gehäuseinnere einbringbar ist. Durch eine solche konstruktive Maßnahme kann das Drehelement zumindest im Wesentlichen in Umfangsrichtung mit Druckluft beaufschlagt werden, wodurch sich die Effektivität der Vorrichtung erheblich verbessert.

Weiterhin ist denkbar, dass das Drehelement form- und/oder kraftschlüssig mit der inneren Röhre verbunden ist. Das Drehelement kann beispielsweise auf die innere Röhre aufgeschoben bzw. mit dieser verpresst sein. Das Drehelement und die innere Röhre können miteinander korrespondierende, in Axialrichtung und/oder in Umfangsrichtung verlaufende Ausnehmungen und Vertiefungen aufweisen, um eine formschlüssige Verbindung zu realisieren.

Zur Erzeugung der Drehbewegung weist das Drehelement wenigsten einen sich zumindest im Wesentlichen radial nach außen erstreckenden Vorsprung auf.

Der Vorsprung dient zur Druckluftaufnahme bzw. Druckluftbeaufschlagung, so dass die Druckluft im Wesentlichen auf den Vorsprung auftrifft und dadurch das Drehelement samt innerer Röhre innerhalb der äußeren Röhre dreht. Zur Vergrößerung der Fläche, auf die die Druckluft wirkt, kann der Vorsprung gekrümmt ausgebildet sein.

Gemäß einer weiteren Ausgestaltung ist es denkbar, dass das Drehelement mehrere Rotorblätter zur Erzeugung der Drehbewegung aufweist. Das Drehelement kann folglich als Lüfterrad bzw. Turbine ausgebildet und durch die Beaufschlagung mit Druckluft in Drehung versetzbar sein.

In weiter vorteilhafter Weise kann ein elastisches Rückstellmittel angeordnet sein, so dass das Drehelement bei Wegfall der Beaufschlagung mit Druckluft in eine Grundposition verbringbar ist. Dadurch erfolgt die durch die Beaufschlagung mit Druckluft hervorgerufene Auslenkung bzw. Drehung des Drehelement in Umfangsrichtung und/oder in Axialrichtung gegen die Federkraft.

In besonders vorteilhafter Weise kann das Rückstellmittel als Rotationsfeder ausgebildet sein. Durch die Rotationsfeder kann die innere Röhre in die Ausgangsposition bzw. Ruheposition zurückgedreht werden, wenn keine Druckluft auf das Drehelement wirkt. Somit ist eine alternierende Bewegung des Drehelements und somit der inneren Röhre möglich, indem nämlich das Drehelement mit kurzen Druckluftimpulsen ausgelenkt und durch die Rotationsfeder wieder zurückgedreht wird. Des Weiteren kann das Rückstellmittel als Feder, insbesondere als Stauchfeder, ausgebildet sein. Eine solche Konstruktion eignet sich besonders, wenn das Drehelement bei Druckluftbeaufschlagung neben einer Drehbewegung zumindest geringfügig auch eine Bewegung in Axialrichtung ausführt, somit entgegen der Federkraft. Sobald die Druckluftbeaufschlagung endet, wird das Drehelement von der Feder in Axialrichtung hin zu dem proximalen Ende der Vorrichtung gedrückt und somit abgebremst.

In besonders vorteilhafter Weise kann das Rückstellmittel derart realisiert sein, dass es mit einem Ende in eine Gehäuseausnehmung und mit dem anderen Ende in eine Drehelementausnehmung eingreift. Diese Ausgestaltung eignet sich besonders für die Anordnung einer Rotationsfeder.

Um das Drehelement besonders zuverlässig mit Druckluft beaufschlagen zu können, kann zwischen dem Gehäusedeckel und dem Gehäuse ein Abdichtmittel, insbesondere ein O-Ring, angeordnet sein. Alternativ oder zusätzlich ist denkbar, dass ein Anschlag am Innenrohr, Außenrohr und/oder an einem weiteren Bauteil vorgesehen ist, sodass die Drehbewegung des Innenrohrs kleiner oder gleich 180° ist. In weiter vorteilhafter Weise kann die Innenschneidkante halbiert oder geöffnet sein.

Die Erfindung wird durch den unabhängigen Anspruch 1 definiert.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1a, 1b: jeweils in einer schematischen, vergrößerten Darstellung das distale Ende einer erfindungsgemäßen Vorrichtung
- Fig. 2: in einer schematischen Darstellung ein Ausführungsbeispiel einer erfindungsgemäßen Vorrichtung,
- Fig. 3: in einer weiteren schematischen, geschnittenen Darstellung das Ausführungsbeispiel gemäß Fig. 2,
- Fig. 4: in einer vergrößerten, geschnittenen Darstellung einen Ausschnitt aus dem Ausführungsbeispiel gemäß Fig. 2,
- Fig. 5: in einer schematischen Darstellung, das Drehelement des Ausführungsbeispiels gemäß Fig. 2.
- Fig. 6: in einer weiteren Darstellung das Drehelement aus Fig. 5,
- Fig. 7: in einer weiteren Darstellung das Drehelement aus Fig. 5,
- Fig. 8a, 8b: jeweils in einer schematischen, perspektivischen Darstellung den Gehäusedeckel der Vorrichtung aus Fig. 2,
- Fig. 9a, 9b: jeweils in einer schematischen, perspektivischen Darstellung den Gehäusedeckel der Vorrichtung aus Fig. 2,
- Fig. 10: in einer schematischen Darstellung ein weiteres Ausführungsbeispiel einer erfindungsgemäßen Vorrichtung,
- Fig. 11: in einer weiteren schematischen Darstellung die erfindungsgemäße Vorrichtung aus Fig. 10,
- Fig. 12: in einer vergrößerten, geschnittenen Darstellung einen Ausschnitt aus dem Ausführungsbeispiel gemäß Fig. 10,
- Fig. 13: in einer schematischen, perspektivischen Darstellung das Rückstellmittel der Vorrichtung gemäß Fig. 10,
- Fig. 14a, 14b: jeweils in einer schematischen, perspektivischen Darstellung das Drehelement der Vorrichtung aus Fig. 10,
- Fig. 15a, 15b: jeweils in einer schematischen, perspektivischen Darstellung das Drehelement der Vorrichtung aus Fig. 10, und
- Fig. 16a, 16b: jeweils in einer schematischen, perspektivischen Darstellung den Gehäusedeckel der Vorrichtung aus Fig. 10.

An dieser Stelle wird zunächst darauf hingewiesen, dass zur Verbesserung der Übersichtlichkeit in einigen Figuren nicht jedes Element mit einem Bezugszeichen versehen ist. Des Weiteren werden in den Figuren gleiche Elemente mit gleichen Bezugszeichen versehen.

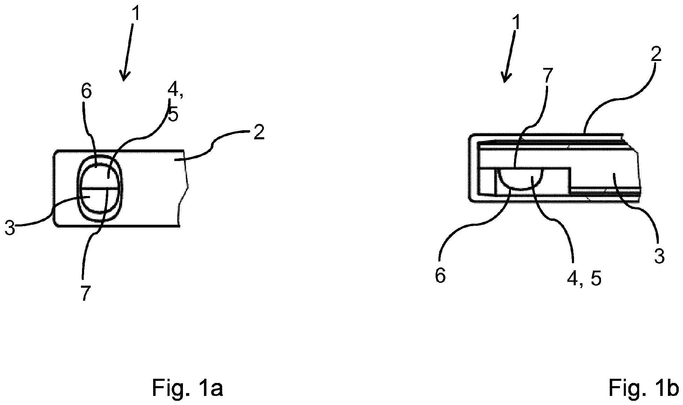

In den Figuren 1a und 1b ist jeweils der Bereich des distalen Endes 1 einer erfindungsgemäßen Vorrichtung zum Schneiden und Absaugen von Gewebe aus dem Auge, insbesondere zum Einsatz in der Vitrektomie, gezeigt. Darin ist deutlich zu erkennen, dass die Vorrichtung eine äußere Röhre 2 und eine in der äußeren Röhre 2 drehbar angeordnete innere Röhre 3 aufweist. Die äußere Röhre 2 umfasst eine seitliche Öffnung 4 und die innere Röhre 3 umfasst eine seitliche Öffnung 5. An der seitlichen Öffnung 4 ist zumindest eine Außenschneidkante 6 und an der seitlichen Öffnung 5 ist zumindest eine Innenschneidkante 7 ausgebildet. Die Außenschneidkante 6 und die Innenschneidkante 7 wirken zusammen, nämlich beim Drehen der inneren Röhre 3 gegenüber der äußeren Röhre 2. Das abgetrennte Gewebe wird mittels Unterdruck durch die innere Röhre 2 aus dem Auge abgesaugt. Es ist von möglich und von Vorteil, mehrere seitliche Öffnungen 4, 5 vorzusehen, beispielsweise zwei seitliche Öffnungen 4, 5. Dadurch erhöht sich auch die Anzahl der Außenschneidkanten 6 und Innenschneidkanten 7, so dass die Leistungsfähigkeit der Vorrichtung verbessert wird.

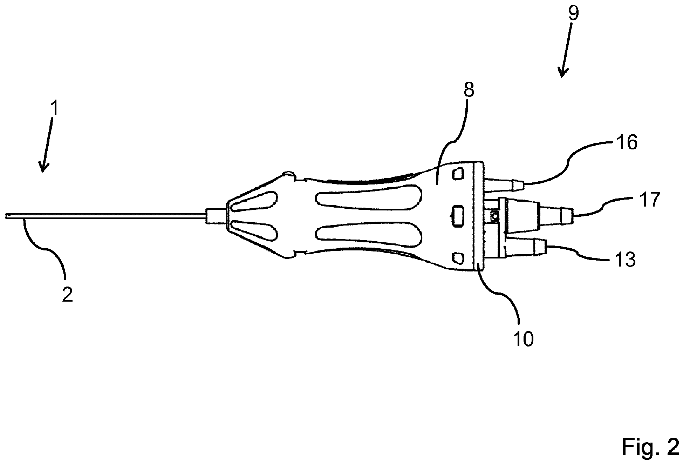

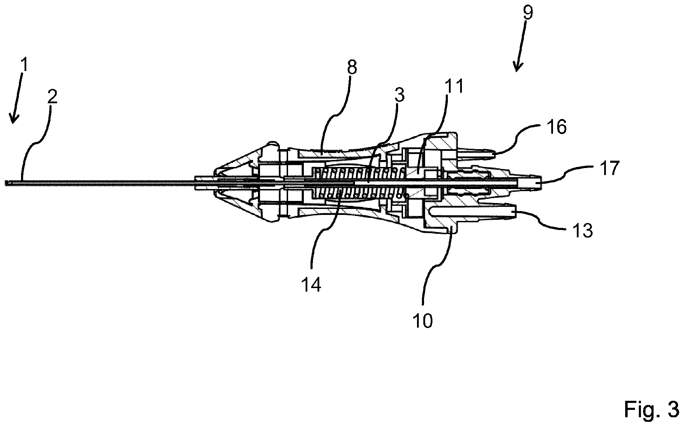

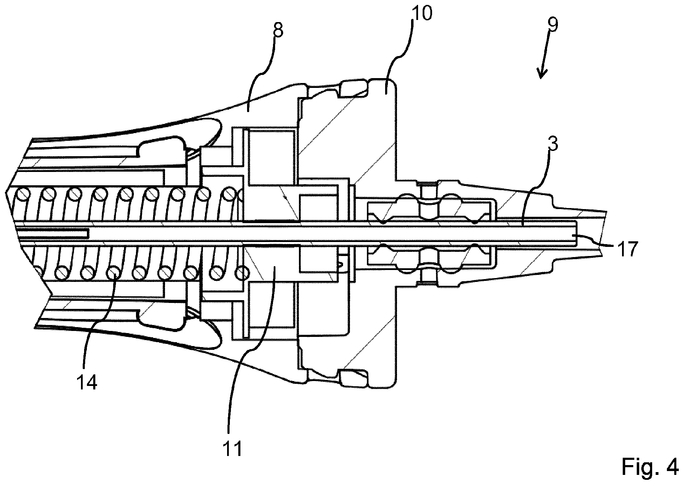

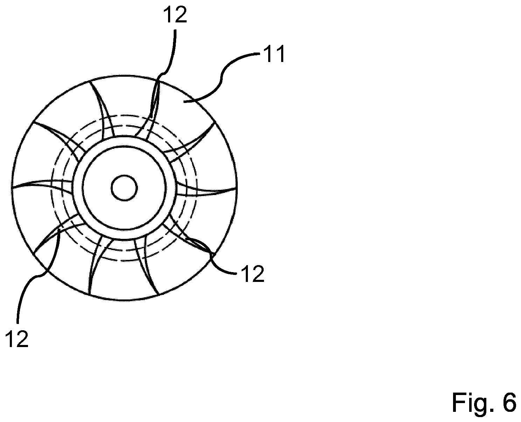

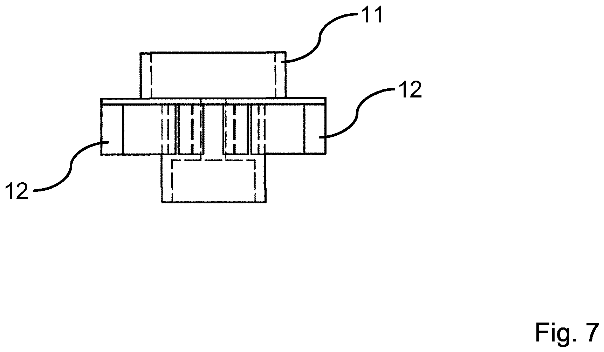

In den Figuren 2 bis 9 sind ein Ausführungsbeispiel einer erfindungsgemäßen Vorrichtung sowie einzelne Elemente dieser Vorrichtung dargestellt. Die Vorrichtung weist eine äußere Röhre 2 auf, in der eine innere Röhre 3 drehbar angeordnet ist, so dass die Außenschneidkante 6 bzw. die Außenschneidkanten 6 mit der Innenschneidkante 7 bzw. den Innenschneidkanten 7 schneidend zusammenwirken. Die innere Röhre 2 und die äußere Röhre 3 sind zumindest bereichsweise in einem Gehäuse 8 angeordnet, das an dem proximalen Ende 9 mit einem Gehäusedeckel 10 verschlossen ist.

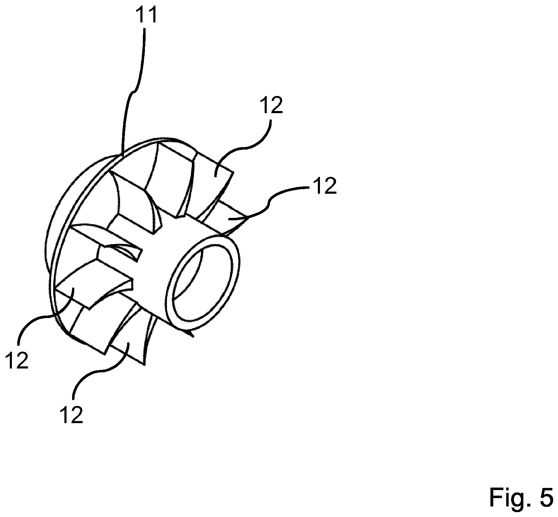

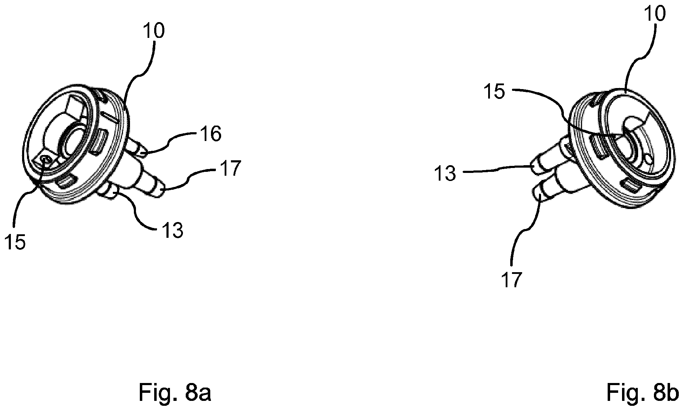

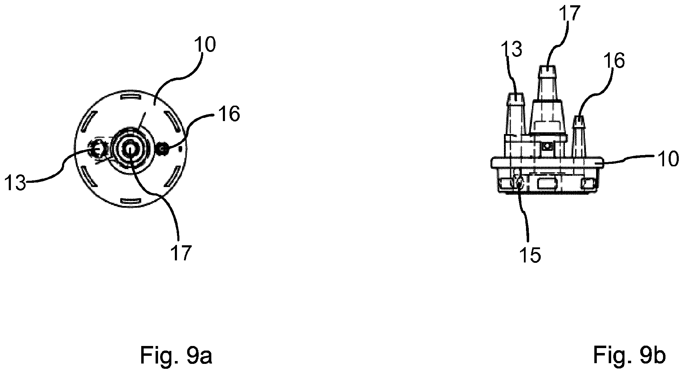

Die innere Röhre 3 ist mit einem Drehelement 11 gekoppelt, das unter Beaufschlagung mit Druckluft eine Drehbewegung ausführt, so dass damit auch die innere Röhre 3 gegenüber der äußeren Röhre 2 gedreht wird. Insbesondere den Figuren 5 bis 7 ist zu entnehmen, dass den das Drehelement 11 Rotorblätter 12 aufweist, somit als Turbinenrad bzw. Lüfterrad realisiert ist. Des Weiteren ist insbesondere in den Fig. 8a, 8b, 9a und 9b gezeigt, dass an dem Gehäusedeckel 10 ein Durchgang 13 ausgebildet ist, über das Drehelement 11 mit Druckluft beaufschlagbar ist. Durch die Druckluft wird das Drehelement 11 gegen das als Feder bzw. Stauchungsfeder ausgebildete Rückstellelement 14 nach vorne gedrückt und gleichzeitig in Drehung versetzt. Im drucklosen Zustand drückt das Rückstellelement 14 das Drehelement 11 zurück gegen den Gehäusedeckel 10. Dadurch wird das Drehelement 11 gebremst und kommt schließlich in seiner Grundposition zum Stehen. Die Abdichtung zwischen Gehäusedeckel 10 und Gehäuse 8 kann über ein in den Fig. 2 bis 9 nicht dargestelltes Abdichtmittel, beispielsweise einen O-Ring, erfolgen.

Die Öffnung 15 des Durchgangs 13 mündet in Umfangsrichtung in das Gehäuseinnere, so dass die Druckluft im Wesentlichen in Umfangsrichtung auf das Drehelement 11 bzw. dessen Rotorblätter 12 wirkt. Dadurch wird die Effektivität der Vorrichtung erheblich verbessert.

Um das von dem Gehäusedeckel 10 eingeschlossene Volumen gezielt zu entlüften, ist ein Entlüftungskanal 16 vorgesehen. Des Weiteren kann abgetrenntes Gewebe über einen in dem Gehäusedeckel 10 ausgebildeten Aspirationskanal 17 über die innere Röhre 3 abgesaugt werden.

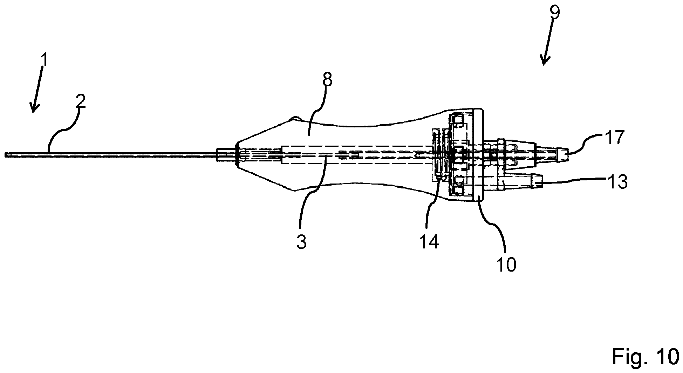

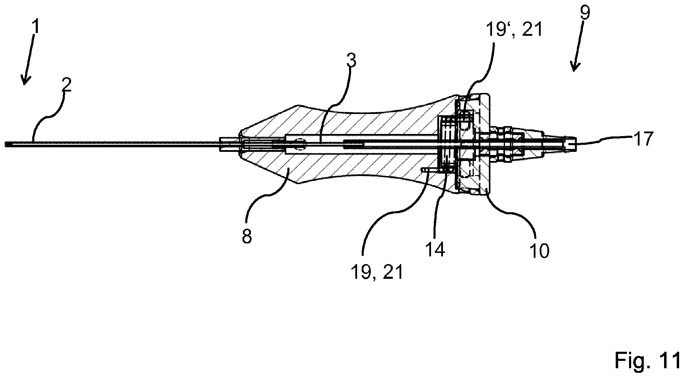

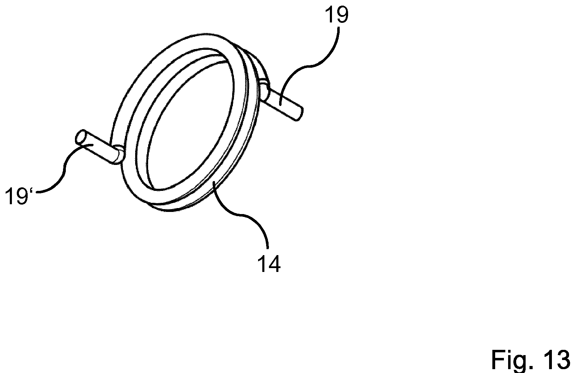

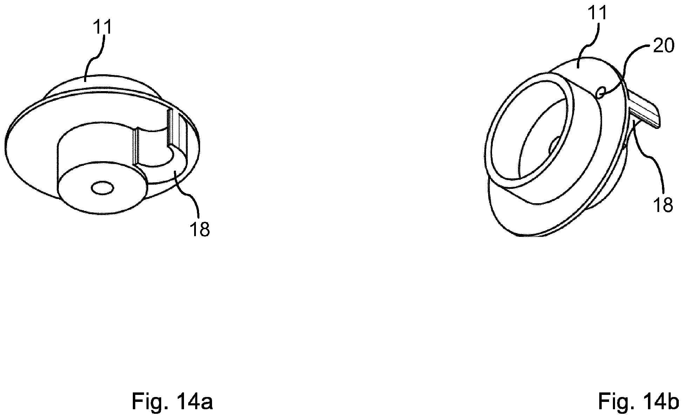

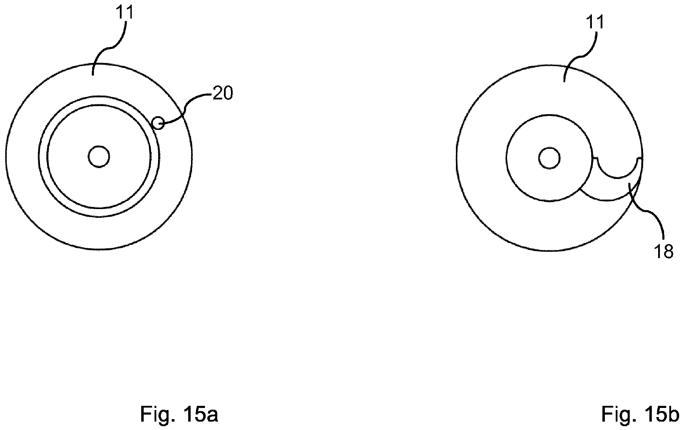

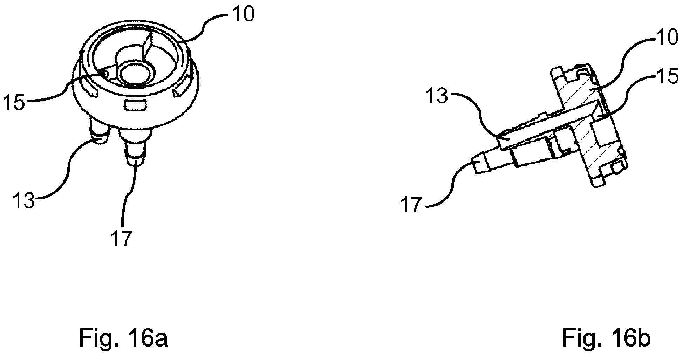

In den Figuren 10 bis 16 ist ein weiteres Ausführungsbeispiel einer erfindungsgemäßen Vorrichtung gezeigt. Dabei sind die zu dem Ausführungsbeispiel gemäß den Figuren 2 bis 9 analogen Elemente mit den gleichen Bezugszeichen versehen.

Das zweite Ausführungsbeispiel unterscheidet sich von der Vorrichtung gemäß Fig. 2 bis 9 im Wesentlichen durch die Ausgestaltung des Drehelements 11 und des Rückstellmittels 14. Das Drehelement 11 weist einen sich radial erstreckenden Vorsprung 18 auf, der zur Beaufschlagung mit Druckluft dient. Der Vorsprung 18 ist gekrümmt ausgebildet, so dass die mit Druckluft zu beaufschlagende Fläche vergrößert ist. Dabei ist denkbar, dass mehrere Vorsprünge 18 vorgesehen sind.

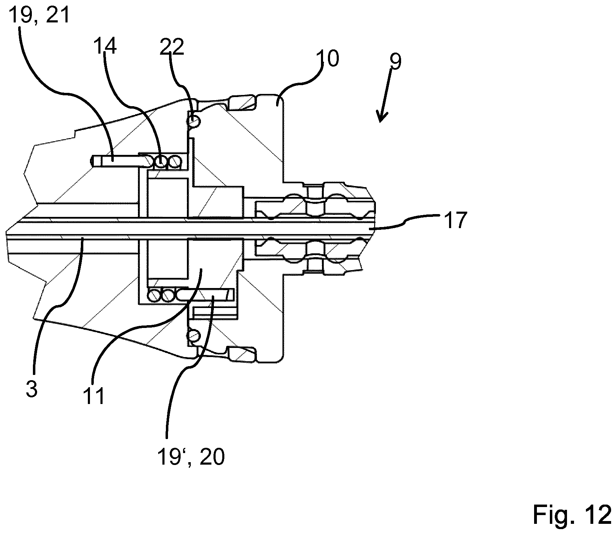

Das Rückstellmittel 14 ist als Rotationsfeder ausgebildet, die mit ihren Enden 19, 19' in eine Drehelementausnehmung 20 und in eine Gehäuseausnehmung 21 eingreift. Wird über den Durchgang 13 des Gehäusedeckels 10 ein Druckluftstoß in das Gehäuseinnere eingebracht, wird das Drehelement 11 gegen die Kraft des Rückstellmittels 14 eine Drehbewegung ausführen. Sobald keine Druckluft mehr auf das Drehelement 11 bzw. dessen Vorsprung 18 wirkt, dreht das Rückstellmittel 14 das Drehelement 11 zurück in die Grundposition. Im Gegensatz zu dem Ausführungsbeispiel gemäß Fig. 2 bis 9 führen das Drehelement 11 und somit die innere Röhre 3 eine alternierende Bewegung aus. Dadurch ist es denkbar, dass die seitlichen Öffnungen 4, 5 und die daran ausgebildeten Außenschneidkanten 6 und Innenschneidkanten 7 derart realisiert sind, dass auch bei der von dem Rückstellmittel 14 hervorgerufenen Bewegung Gewebe geschnitten wird. Insbesondere Fig. 12 ist zu entnehmen, dass ein als O-Ring ausgebildetes Abdichtmittel 22 zwischen dem Gehäusedeckel 10 und dem Gehäuse ausgebildet ist.

Des Weiteren entspricht die Vorrichtung gemäß Figuren 10 bis 16 der Vorrichtung gemäß den Figuren 2 bis 9, so dass auf die diesbezügliche Beschreibung verwiesen wird.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: distales Ende
- 2: äußere Röhre
- 3: innere Röhre
- 4: seitliche Öffnung (äußere Röhre)
- 5: seitliche Öffnung (innere Röhre)
- 6: Außenschneidkante
- 7: Innenschneidkante
- 8: Gehäuse
- 9: proximales Ende
- 10: Gehäusedeckel
- 11: Drehelement
- 12: Rotorblätter
- 13: Durchgang
- 14: Rückstellmittel
- 15: Öffnung
- 16: Entlüftungskanal
- 17: Aspirationskanal
- 18: Vorsprung
- 19', 19: Enden
- 20: Drehelementausnehmung
- 21: Gehäuseausnehmung
- 22: Abdichtmittel

## Patentansprüche

1. Vorrichtung zum Schneiden und Absaugen von Gewebe aus dem menschlichen oder tierischen Auge, insbesondere zur Durchführung einer Vitrektomie oder eines Netzhautpeelings, mit einer äußeren Röhre (2) und einer in der äußeren Röhre (2) drehbaren inneren Röhre (3), wobei die äußere Röhre (2) wenigstens eine seitliche Öffnung (4) mit zumindest einer Außenschneidkante (6) aufweist, wobei die innere Röhre (3) mindestens eine seitliche Öffnung (5) mit zumindest einer Innenschneidkante (7) aufweist, wobei die Innenschneidkante (7) und die Außenschneidkante (6) beim Drehen der inneren Röhre (3) gegenüber der äußeren Röhre (2) schneidend zusammen wirken, wobei
die innere Röhre (3) mittelbar oder unmittelbar mit einem Drehelement (11) verbunden ist und wobei das Drehelement (11) und somit die innere Röhre (3) bei Beaufschlagung mit Druckluft eine Drehbewegung ausführen,
**dadurch gekennzeichnet, dass** das Drehelement (11) einen sich zumindest bereichsweise radial erstreckenden Vorsprung (18) zur Beaufschlagung mit Druckluft aufweist.

2. Vorrichtung nach Anspruch 1, **dadurch gekennzeichnet, dass** die innere Röhre (3) und die äußere Röhre (2) zumindest bereichsweise in und/oder an einem Gehäuse (8) angeordnet sind und dass das Gehäuse (8) am proximalen Ende (9) mit einem Gehäuseboden oder mit einem Gehäusedeckel (10) verschlossen ist.

3. Vorrichtung nach Anspruch 2, **dadurch gekennzeichnet, dass** das Gehäuse (8) bzw. der Gehäuseboden und/oder der Gehäusedeckel (10) mindestens einen Durchgang (13) zur Beaufschlagung des Drehelements (11) mit Druckluft aufweist.

4. Vorrichtung nach Anspruch 3, **dadurch gekennzeichnet, dass** der Durchgang (13) derart ausgebildet ist, dass die Druckluft im Wesentlichen in Umfangsrichtung in das Gehäuseinnere einbringbar ist.

5. Vorrichtung nach einem der Ansprüche 1 bis 4, **dadurch gekennzeichnet, dass** das Drehelement (11) form- und/oder kraftschlüssig mit der inneren Röhre (3) verbunden ist.

6. Vorrichtung nach einem der Ansprüche 1 bis 5, **dadurch gekennzeichnet, dass** der Vorsprung (18) gekrümmt ausgebildet ist.

7. Vorrichtung nach einem der Ansprüche 1 bis 6, **dadurch gekennzeichnet, dass** ein elastisches Rückstellmittel (14) angeordnet ist, so dass das Drehelement (11) bei Wegfall der Druckluftbeaufschlagung in eine Grundposition verbringbar ist.

8. Vorrichtung nach Anspruch 7, **dadurch gekennzeichnet, dass** das Rückstellmittel (14) als Feder, insbesondere als Rotationsfeder und/oder als Stauchungsfeder, ausgebildet ist.

9. Vorrichtung nach Anspruch 7 oder 8, **dadurch gekennzeichnet, dass** das Rückstellmittel (14) mit einem Ende (19, 19') in eine Gehäuseausnehmung (21) und mit einem anderen Ende (19, 19') in eine Drehelementausnehmung (20) eingreift.

10. Vorrichtung nach Anspruch 9, **dadurch gekennzeichnet, dass** zwischen dem Gehäusedeckel (10) und dem Gehäuse (8) ein Abdichtmittel (22), insbesondere ein O-Ring, angeordnet ist.

## Claims

1. Device for cutting and drawing off tissue from the human or animal eye, in particular for carrying out a vitrectomy or retina peeling, having an outer tube (2) and an inner tube (3) which can be rotated in the outer tube (2), wherein the outer tube (2) has at least one lateral opening (4) having at least one outer cutting edge (6), wherein the inner tube (3) has at least one lateral opening (5) having at least one inner cutting edge (7), wherein the inner cutting edge (7) and the outer cutting edge (6) cooperate in a cutting manner with each other when the inner tube (3) is rotated relative to the outer tube (2), wherein
the inner tube (3) is directly or indirectly connected to a rotary element (11) and wherein the rotary element (11) and consequently the inner tube (3) carry out a rotational movement when compressed air is applied,
**characterised in that** the rotary element (11) has an at least partially radially extending projection (18) for actuation with compressed air.

2. Device according to claim 1, **characterised in that** the inner tube (3) and the outer tube (2) are arranged at least partially in and/or on a housing (8) and **in that** the housing (8) is locked at the proximal end (9) with a housing base or with a housing cover (10).

3. Device according to claim 2, **characterised in that** the housing (8) or the housing base and/or the housing cover (10) has at least one passage (13) for acting on the rotary element (11) with compressed air.

4. Device according to claim 3, **characterised in that** the passage (13) is constructed in such a manner that the compressed air can be introduced substantially in a peripheral direction into the housing interior.

5. Device according to any one of claims 1 to 4, **characterised in that** the rotary element (11) is connected to the inner tube (3) in a positive-locking and/or non-positive-locking manner.

6. Device according to any one of claims 1 to 5, **characterised in that** the projection (18) is constructed in a curved manner.

7. Device according to any one of claims 1 to 6, **characterised in that** a resilient return means (14) is arranged so that the rotary element (11) can be moved into a basic position when the application of compressed air is discontinued.

8. Device according to claim 7, **characterised in that** the restoring means (14) is constructed as a spring, in particular as a rotary spring and/or as a compression spring.

9. Device according to claim 7 or 8, **characterised in that** the restoring means (14) engages with one end (19, 19') in a housing recess (21) and with another end (19, 19') in a rotary element recess (20).

10. Device according to claim 9, **characterised in that** a sealing means (22), in particular an O-ring, is arranged between the housing cover (10) and the housing (8).

## Revendications

1. Dispositif pour la découpe et l'aspiration d'un tissu de l'œil humain ou animal, plus particulièrement pour la réalisation d'une vitrectomie ou d'un pelage de la rétine, avec un tube externe (2) et un tube interne (3) rotatif dans le tube externe (2), dans lequel le tube externe (2) comprend au moins une ouverture latérale (4) avec au moins une arête de coupe externe (6), dans lequel le tube interne (3) comprend au moins une ouverture latérale (5) avec au moins une arête de coupe interne (7), dans lequel l'arête de coupe interne (7) et l'arête de coupe externe (6) interagissent de manière coupante lors de la rotation du tube interne (3) par rapport au tube externe (2), dans lequel
le tube interne (3) est relié indirectement ou directement avec un élément rotatif (11) et dans lequel l'élément rotatif (11), et donc le tube interne (3), effectue un mouvement de rotation lors d'une sollicitation avec de l'air comprimé,
**caractérisé en ce que** l'élément rotatif (11) comprend une saillie (18) s'étend radialement au moins à certains endroits, pour la sollicitation avec de l'air comprimé.

2. Dispositif selon la revendication 1, **caractérisé en ce que** le tube interne (3) et le tube externe (2) sont disposés, au moins à certains endroits dans et/ou sur un boîtier (8) et **en ce que** le boîtier (8) est fermé, à l'extrémité proximale (9), avec un fond de boîtier ou avec un couvercle de boîtier (10).

3. Dispositif selon la revendication 2, **caractérisé en ce que** le boîtier (8) resp. le fond de boîtier et/ou le couvercle de boîtier (10) comprend au moins un passage (13) pour la sollicitation de l'élément rotatif (11) avec de l'air comprimé.

4. Dispositif selon la revendication 3, **caractérisé en ce que** le passage (13) est conçu de façon à ce que l'air comprimé peut être introduit globalement, dans la direction de la circonférence, à l'intérieur du boîtier.

5. Dispositif selon l'une des revendications 1 à 4, **caractérisé en ce que** l'élément rotatif (11) est relié par complémentarité de forme et/ou par force avec le tube interne (3).

6. Dispositif selon l'une des revendications 1 à 5, **caractérisé en ce que** la saillie (18) est incurvée.

7. Dispositif selon l'une des revendications 1 à 6, **caractérisé en ce qu'**un moyen de rappel élastique (14) est disposé de façon à ce que l'élément rotatif (11) puisse être amené dans une position de base en l'absence de sollicitation par de l'air comprimé.

8. Dispositif selon la revendication 7, **caractérisé en ce que** le moyen de rappel (14) est conçu comme un ressort, plus particulièrement comme un ressort à rotation et/ou comme un ressort à compression.

9. Dispositif selon la revendication 7 ou 8, **caractérisé en ce que** le moyen de rappel (14) s'emboîte, avec une extrémité (19, 19'), dans un évidement du boîtier (21) et, avec une autre extrémité (19, 19'), dans un évidement de l'élément rotatif (20).

10. Dispositif selon la revendication 9, **caractérisé en ce que**, entre le couvercle du boîtier (10) et le boîtier (8) est disposé un moyen d'étanchéité (22), plus particulièrement un joint torique.
